(19) Europäisches Patentamt
European Patent Office
Office européen des brevets

(11) Publication number: **0 277 743**
**A1**

# (12) EUROPEAN PATENT APPLICATION

(21) Application number: **88300609.0**

(51) Int. Cl.⁴: **C07D 487/04**, A61K 31/40, //(C07D487/04,209:00,205:00)

(22) Date of filing: **26.01.88**

(30) Priority: **02.02.87 US 9865**

(43) Date of publication of application:
**10.08.88 Bulletin 88/32**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Salzmann, Thomas N.**
**154 Medowbrook Drive**
**North Plainfield New Jersey 07062(US)**
Inventor: **Dininno, Frank P.**
**5 Benjamin Court**
**Old Bridge New Jersey 08857(US)**
Inventor: **Muthard, David A.**
**27 Ticonderoga Blvd.**
**Freehold New Jersey 07728(US)**

(74) Representative: **Hesketh, Alan, Dr.**
**European Patent Department Merck & Co.,**
**Inc. Terlings Park Eastwick Road**
**Harlow Essex, CM20 2QR(GB)**

## (54) 2-(heteroaryliumalkyl)phenyl carbapenem antibacterial agents.

(57) Carbapenems having the formula:

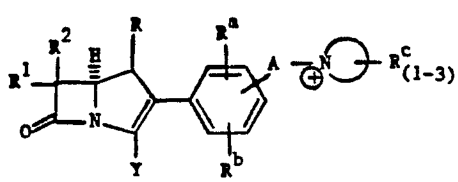

(I.)

are useful antibacterial agents.

EP 0 277 743 A1

# 2-(HETEROARYLIUMALKYL)PHENYL CARBAPENEM ANTIBACTERIAL AGENTS

## BACKGROUND OF THE INVENTION

The present invention relates to antibacterial agents of the carbapenem class, in which the 2-position sidechain is characterized by a phenyl moiety, optionally substituted, to which is attached, usually through an alkyl bridge, a nitrogen-containing heterocycle, with attachment being through the nitrogen atom, as described in more detail further below.

Thienamycin was an early carbapenem antibacterial agent having a broad spectrum; it has the following formula:

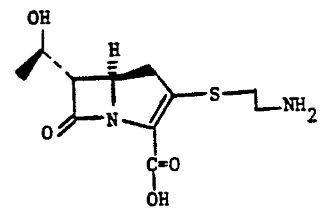

Later, N-formimidoyl thienamycin was discovered; it has the formula:

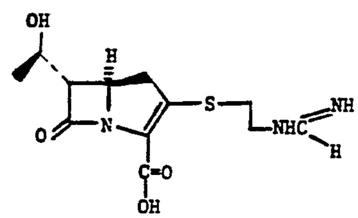

The 2-(heteroaryliumalkyl)phenyl carbapenems of the present invention have an antibacterial spectrum and potency equal to or greater than, in most cases, that of either thienamycin or N-formimidoyl thienamycin. The compounds of the present invention are also more resistant than thienamycin or N-formimidoyl thienamycin to degradation by the dehydropeptidase enzyme DHP-I, thus permitting greater therapeutic application of the compounds.

More recently, carbapenem antibacterial agents have been described which have a 2-substituent which is an aryl moiety optionally substituted by, e.g., aminomethyl and substituted aminomethyl. These agents are described in U.S. Pat. Nos. 4,543,257 and 4,260,627 and have the formula:

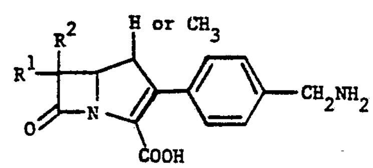

However, these compounds belong to a different class from those of the present invention and are distinguished by different antibacterial properties.

SUMMARY OF THE INVENTION

The present invention provides novel carbapenem compounds of the formula:

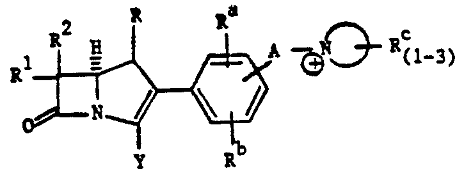

(I.)

wherein:

R is H or $CH_3$;

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$__, $FCH_2$-, $F_2CH$-, $F_3C$-, $CH_3CH(F)$-, $CH_3CF_2$-, or $(CH_3)_2C(F)$-;

$R^a$, $R^b$ and $R^c$ ($R^c$ represents from 1 to 3 substituents which may be the same or different) are independently selected from the group consisting of:

a) a trifluoromethyl group: $-CF_3$;

b) a halogen atom: -Br, -Cl, -F, or -I;

c) $C_1$-$C_4$ alkoxy radical: $-OC_{1-4}$ alkyl;

d) a hydroxy group: -OH;

e) ($C_1$-$C_6$ alkyl) carbonyloxy radical:

$-O\overset{O}{\overset{\|}{C}}\,C_{1-6}$ alkyl;

f) a carbamoyloxy radical which is unsubstituted or substituted on nitrogen with one or two $C_1$-$C_4$ alkyl groups:

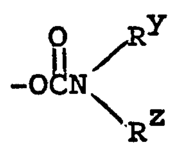

where $R^y$ and $R^z$ are independently H or $C_{1-4}$ alkyl;

g) a $C_1$-$C_6$ alkylthio radical, $C_1$-$C_6$ alkylsulfinyl radical or $C_1$-$C_6$ alkylsulfonyl radical:

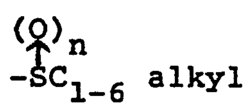

where n = 0-2, and the alkyl portion is optionally substituted by cyano;

h) a sulfamoyl group which is unsubstituted or substituted on nitrogen by one or two $C_1$-$C_4$ alkyl groups:

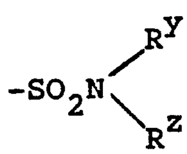

where $R^y$ and $R^z$ are as defined above;

i) an amino group, or a mono ($C_1$-$C_4$ alkyl) amino or di($C_1$-$C_4$ alkyl)-amino group:

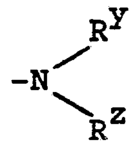

where $R^y$ and $R^z$ are as defined above;

j) a formylamino group:

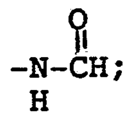

k) ($C_1$-$C_6$ alkyl)carbonylamino radical:

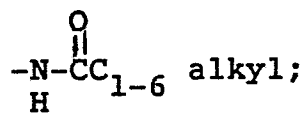

l) a ($C_1$-$C_4$ alkoxy) carbonylamino radical:

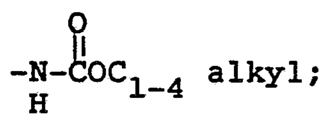

m) a ureido group in which the terminal nitrogen is unsubstituted or substituted with one or two $C_1$-$C_4$ alkyl groups:

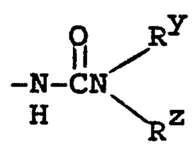

where $R^y$ and $R^z$ are as defined above;

n) a sulfonamido group: $-\underset{H}{N}SO_2$;

o) a cyano group: -CN;

p) a formyl or acetalized formyl radical:

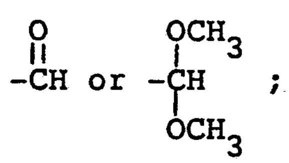

q) ($C_1$-$C_6$ alkyl)carbonyl radical wherein the carbonyl is free or acetalized:

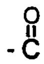 $C_{1-6}$ alkyl or

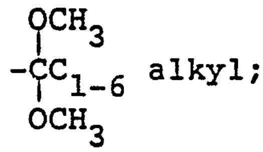

r) phenylcarbonyl:

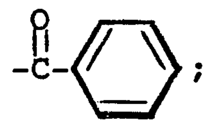

s) a hydroximinomethyl radical in which the oxygen or carbon atom is optionally substituted by a $C_1$-$C_4$ alkyl group:

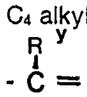 $= NOR^z$ where $R^y$ and $R^z$ are as defined above;

t) a ($C_1$-$C_6$ alkoxy)carbonyl radical:

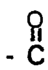 $OC_{1-6}$ alkyl;

u) a carbamoyl radical which is unsubstituted or substituted on nitrogen by one or two $C_1$-$C_4$ alkyl groups:

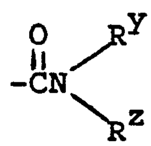

where $R^y$ and $R^z$ are as defined above;

v) an N-hydroxycarbamoyl or N($C_1$-$C_4$ alkoxy)carbamoyl radical in which the nitrogen atom may be additionally substituted by a $C_1$-$C_4$ alkyl group:

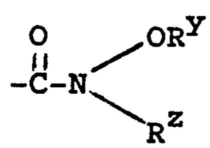

where $R^y$ and $R^z$ are as defined above;

w) a thiocarbamoyl group: 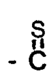 $NH_2$;

x) an amidino group

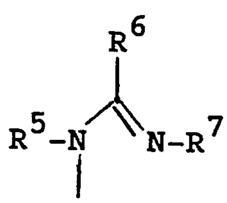

or

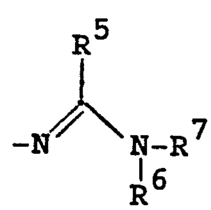

where $R^5$, $R^6$ and $R^7$ are independently hydrogen, $C_1$-$C_4$alkyl or wherein two of the alkyl groups together form a $C_2$-$C_6$alkylidene radical optionally interrupted by a heteroatom and joined together to form a ring;

y) a carboxamidino group

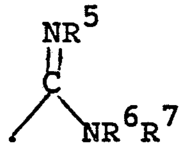

where $R^5$, $R^6$ and $R^7$ are as defined above;

z) a guanidinyl group where $R^6$ in y) above is $NR^8R^9$ and $R^8$ and $R^9$ are as defined for $R^5$ through $R^7$ above;

aa) hydrogen;

ab) $C_2$-$C_6$ alkenyl radical;

ac) $C_2$-$C_6$ alkynyl radical;

ad) $C_3$-$C_7$ cycloalkyl radical;

ae) $C_3$-$C_7$ cycloalkyl methyl radical;

af) $C_5$-$C_7$ cycloalkenyl radical;

ag) phenyl;

ah) $C_1$-$C_6$ alkyl radical;

ai) $C_1$-$C_4$ alkyl monosubstituted by one of the substituents a) - ag) above;

aj) an acidic sidechain of the structure -B or $-(CH_2)_n-X-(CH_2)_m-W-B$

where:

n is 0-4;

m is 0-4;

X is $CR^sR^t$; CH = CH; phenylene ($-C_6H_4-$); NH; N($C_1$-$C_4$ alkyl); O; S; S = O; C = O; $SO_2$; $SO_2NH$; $CO_2$; CONH; $OCO_2$; OC = O; or NHC = O;

where $R^t$ is H or $C_1$-$C_4$-alkyl; and $R^s$ is H, OH, $C_1$-$C_4$-alkyl, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, or $SO_2NH$($C_1$-$C_4$ alkyl);

W is a single bond; NH; N($C_1$-$C_4$ alkyl); O; or S;

B is an acidic function selected from carboxy ($CO_2H$); phosphono [P = O(OH)$_2$]; alkylphosphono {P = O(OH)-[O($C_1$-$C_4$ alkyl)]}; alkylphosphinyl [P = O(OH)-($C_1$-$C_4$alkyl)]; phosphoramido [P = O(OH)$NH_2$]; substituted phosphoramido [P = O(OH)NH($C_1$-$C_4$alkyl) and P = O(OH)$NHR^x$]; sulfino ($SO_2H$); sulfo ($SO_3H$); 5-tetrazolyl ($CN_4H$); arylsulfonamido ($SO_2NHR^x$); and acylsulfonamides selected from the structures $CONHSO_2R^x$, $CONHSO_2NH_2$, $CONHSO_2$($C_1$-$C_4$alkyl), $CONHSO_2NH$($C_1$-$C_4$alkyl), $CONHSO_2N$($C_1$-$C_4$alkyl)$_2$, $SO_2NHCO$($C_1$-$C_4$-alkyl), $SO_2NHCONH_2$, $SO_2NHCONH$($C_1$-$C_4$alkyl), $SO_2NHCON$($C_1$-$C_4$alkyl)$_2$, $SO_2NHCOR^x$, $SO_2NHCSNH_2$, $SO_2NHCSNH$($C_1$-$C_4$alkyl), $SO_2NHCSN$($C_1$-$C_4$ alkyl)$_2$, $SO_2NHSO_2R^x$, and $SO_2NHSO_2R^w$,

where $R^x$ is phenyl or heteroaryl; and $R^w$ is $C_1$-$C_4$alkyl, phenyl, heteroaryl, $NH_2$, NH($C_1$-$C_4$ alkyl), N($C_1$-$C_4$ alkyl)$_2$, OH, or O($C_1$-$C_4$alkyl);

where, for both $R^x$ and $R^w$, heteroaryl is a monocyclic aromatic hydrocarbon group having 5 or 6 ring atoms in which from 1 to 4 carbon ring atoms are replaced by heteroatoms selected from N, O, and S, provided that the minimum number of nitrogen heteroatoms present when 2 to 4 total heteroatoms are selected, is equal to the total number of heteroatoms less one;

and where for both $R^w$ and $R^x$ the phenyl and heteroaryl groups may be further mono-or disubstituted by $C_1$-$C_4$alkyl; $C_1$-$C_4$-alkoxy; Br, Cl, or F; $CF_3$; OH; ($C_1$-$C_4$alkyl)carbonyloxy; carbamoyl or carbamoyloxy or sulfamoyl or amino where the nitrogen may be mono-or disubstituted with $C_1$-$C_4$alkyl; $C_1$-$C_4$alkylthio; $C_1$-$C_4$ alkylsulfinyl; $C_1$-$C_4$alkylsulfoxyl; formylamino; ($C_1$-$C_4$alkyl)carbonylamino; ($C_1$-$C_4$alkoxyl)carbonylamino; ureido in which the terminal nitrogen may be substituted by $C_1$-$C_4$alkyl; ($C_1$-$C_4$alkyl)sulfonamido; cyano; ($C_1$-$C_4$alkoxyl)carbonyl; thiocarbamoyl; nitro; carboxyl; formyl; or ($C_1$-$C_4$alkyl)carbonyl;

A is para (p) or meta (m) with respect to the point of attachment of the phenyl ring to the carbapenem nucleus, and is $(CH_2)_m-Q-(CH_2)_n$, where m is 0 to 2 and n is 1 or 2; and Q is a covalent bond; O; S; SO; $SO_2$; NH; or N($C_1$-$C_4$ alkyl);

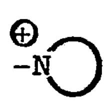

is a monocyclic aromatic hydrocarbon group having 5 or 6 ring atoms in which one of the carbon atoms has been replaced by a nitrogen atom and attachment of said group is by way of said nitrogen atom, and in which one additional carbon atom is optionally replaced by a heteroatom selected from O and S, or from 1 to 3 additional carbon atoms are each optionally replaced by a nitrogen heteroatom; and

Y is selected from:

i) COOH or a pharmaceutically acceptable ester or salt thereof,
ii) $COOR^3$ wherein $R^3$ is a readily removable carboxyl covering group,
iii) COOM wherein M is an alkali metal, or
iv) $COO^{\ominus}$;
provided that when Y is other than iv) a counterion $Z^{\ominus}$ is provided.

The substituent R may be of either configuration, i.e., the α or β-stereoisomer. Neither stereoisomer is preferred, since it has been found that one configuration will prove to be preferable with certain substituents, while with other substituents the opposite configuration gives better results, especially with respect to stability toward the DHP-I enzyme.

The $R^{c}_{(1-3)}$ substituent represents from 1 to 3 substituents which may be the same or different and are selected on an independent basis. While a single such substituent is clearly preferred, there is occasion to use up to three such substituents, e.g., where it is desired to enhance the effect of a particular substituent group by employing multiple substituents. Thus, two carboxymethyl substituents may be used. At other times it may be desired to employ a substituent known to enhance antibacterial activity of the overall molecule against a particular bacterium, for example, while also employing a substituent known to improve the duration of action of the overall molecule.

The overall molecule must be electronically balanced. Since a quaternary nitrogen is always present in the compounds of the present invention, a balancing anion must also be present. This is usually accomplished by having Y be $COO^{\ominus}$. However, where Y is, e.g., a pharmaceutically acceptable ester, a counterion (anion) $Z^{\ominus}$ must be provided, or alternatively, an anionic substituent might be utilized. Further, it is within the scope of this invention to utilize an anionic substituent where the quaternary nitrogen is already balanced by $Y=COO^{\ominus}$. In that case, it will be understood that it is necessary to provide a counterion (cation) for the anionic substituent. However, it is well within the skill of a medicinal chemist, to whom there is available many suitable anionic and cationic counterions, to make such choices.

With reference to the above definitions, "alkyl" means a straight or branched chain aliphatic hydrocarbon radical.

The term "heteroatom" means N, S, or O, selected on an independent basis.

Under the definition of "Y", the term "pharmaceutically acceptable ester or salt" refers to those salt and ester forms of the compounds of the present invention which would be apparent to the pharmaceutical chemist, i.e., those which are non-toxic and which would favorably affect the pharmacokinetic properties of said compounds, their palatability, absorption, distribution, metabolism and excretion. Other factors, more practical in nature, which are also important in the selection, are cost of raw materials, ease of crystallization, yield, stability, hygroscopicity, and flowability of the resulting bulk drug. Since the compounds of the present invention may be carboxylates, the salts would be cations such as benzathine, chloroprocaine, choline, diethanolamine, meglumine and procaine. The metallic cations such as aluminum, calcium, lithium, magnesium and zinc are potential choices. The alkali metal cations sodium and potassium are specifically defined. It will also be noted that the compounds of the present invention are potentially internal salts or zwitterions, since under physiological conditions the carboxyl group may be anionic, and this electronic charge will be balanced off internally against the cationic charge of the heteroarylium group. Where this is not the case, it is provided in the definition of "Y" that a counterion "$Z^{\ominus}$" is present. This counterion is selected from the group of suitable pharmaceutical anions, e.g., chloride, phosphate and tartrate.

The term "readily removable carboxyl covering group" means a conventional substituent which takes the place of the acidic hydrogen of the carboxyl group and thereby prevents said group from reacting with any of the reagents employed in the various steps of the overall synthesis. Such covering of the carboxyl group is often necessary to prevent unwanted competing reactions involving said carboxyl group from taking place. Thus, all of these compounds are intermediates. The conventional covering substituent must also be "readily removable", by which is meant that it is selectively removable, i.e., it is not likely to be removed during the course of ordinary procedures which are to be carried out on the carbapenem nucleus and sidechains, while, on the other hand, it is likely to be removed by procedures which are not so harsh as to disturb the basic ring structure of the carbapenem nucleus or unprotected substituents thereon.

It is preferred that when one of $R^1$ or $R^2$ is H, the other is (R)-$CH_3CH(OH)$-or (R)-$CH_3CH(F)$-, and (R)-$CH_3CH(OH)$-is most preferred.

Representative A groups are $-CH_2-$, $-CH_2CH_2-$, and $-OCH_2CH_2-$. Preferred is $-CH_2-$.

Representative $R^c$ groups are

$-CH_3$,

$-CH_2CH_3$, $-(CH_2)_3CH_3$, $-OCH_3$, $-SCH_3$, 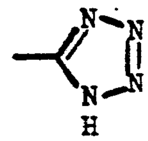 , $-COOH$,

$-NHCH_2COOH$, $-OH$, $-CH_2OH$, $-CH_2COOH$, $-CH_2CH_2COOH$,

$-CH_2CONH_2$, $-CH_2CH_2S^{\oplus}(CH_3)_2$, $-CH_2CH_2SO_3H$, 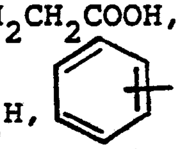 ,
$-CONH_2$, $-SO_2NH_2$, $-SO_3H$, $-NH_2$, $-N(CH_3)_2$, $-CON(CH_3)_2$,
$-NHCH_3$, $-CH_2NH_2$, $-CN$, $-CH_2CN$, $-CH_2SCH_3$, $-CH_2SO_3^{\ominus}$,
$-CH_2SOCH_3$, $-CH_2SO_2CH_3$, $-SO_2CH_3$, $-SOCH_3$, $-CH_2OCH_3$,

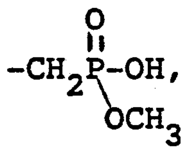, $-CF_3$, $-CH_2O\overset{O}{\overset{\|}{C}}NH_2$, $-CH_2SO_2NH_2$, $-SCH_2CH_2CN$,

Br, Cl, F, $-SCF_3$, $-CH_2SCF_3$, and $-SCH_2CF_3$.

Useful examples of the

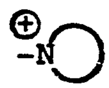

moiety are the following:

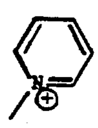

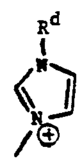

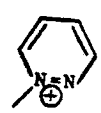

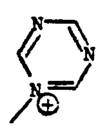

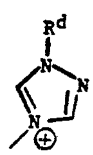

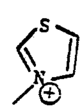

where $R^d$ is $C_1$-$C_4$alkyl, $CH_2SO_3^{\ominus}$ , or $CH_2COR^e$ where $R^e$ is OH, $NH_2$, O($C_1$-$C_4$alkyl), or O-benzyl.

The pyridinium group is preferred since it provides, when incorporated into the carbapenem final product, the desired properties of good antibacterial spectrum and potency combined with chemical stability and satisfactory resistance to hydrolysis by the dihydropeptidase (DHP-I) enzyme. It also provides ready availability and ease of handling as a starting material. However, any of the other groups set out above, as well as those falling within the definition of

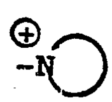

set out herein but not specifically described above, are also suitable, although perhaps in some cases less desirable in terms of one or more of the criteria mentioned above.

For all of the compounds exemplified herein, the R substituent is hydrogen. This is the result of a more facile synthesis for such compounds, however, and does not evidence any preference for R = hydrogen.

To the contrary, the compounds wherein R = methyl will often be found to possess greater stability to dehydropeptidase (DHP-I) enzyme, a renal dipeptidase which mediates lactam hydrolysis of carbapenem compounds in man, resulting in significantly reduced urinary recoveries.

With regard to all of the preferred substituents described above, the following compounds are preferred embodiments of the present invention:

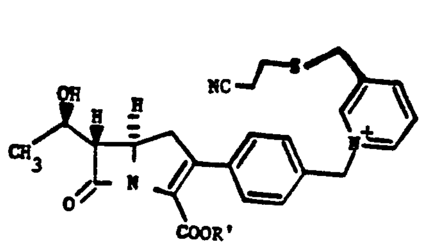

1

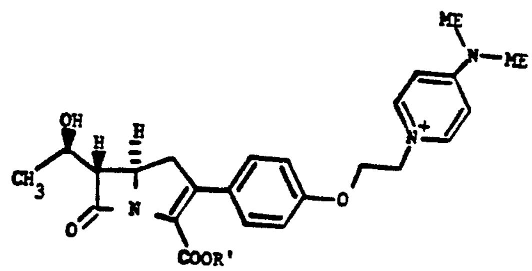

2

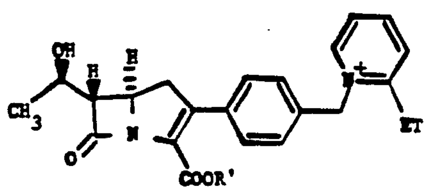

3

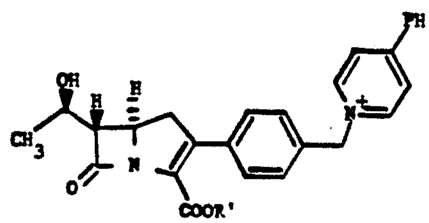

4

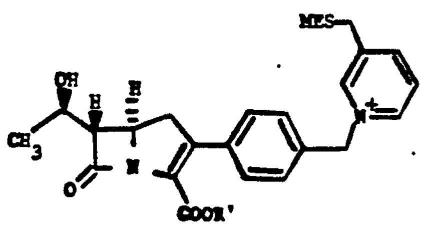

5

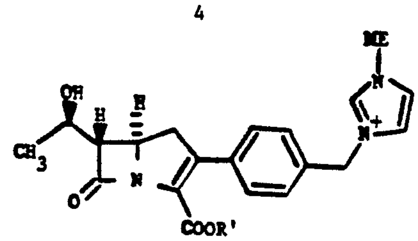

6

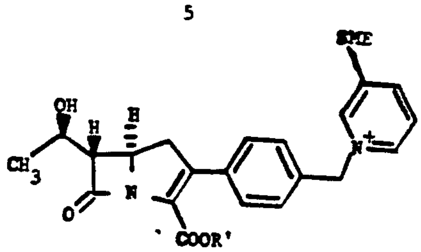

7

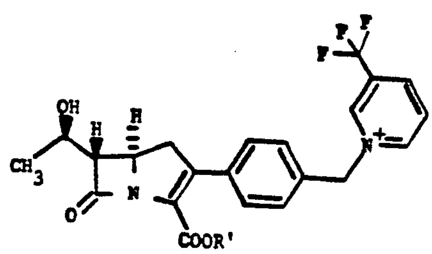

8

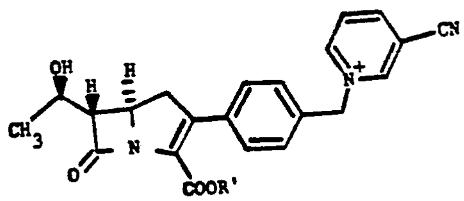

9

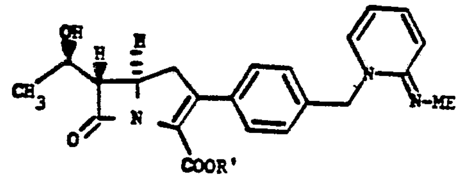

10

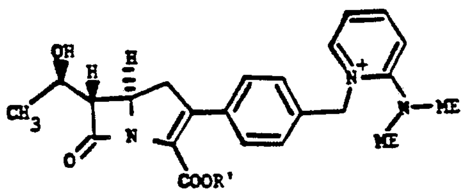

11

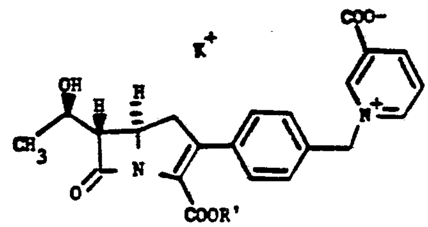

12

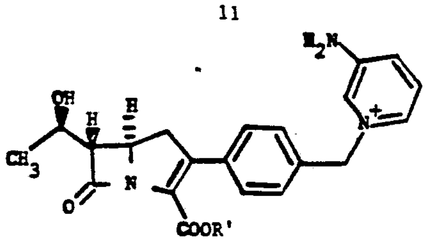

13

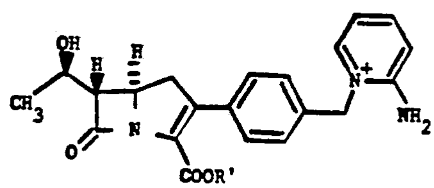

14

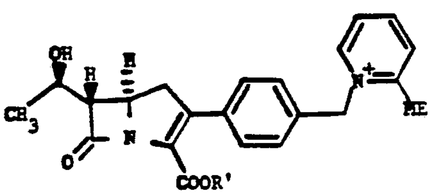

15

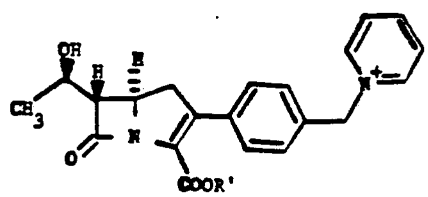

16

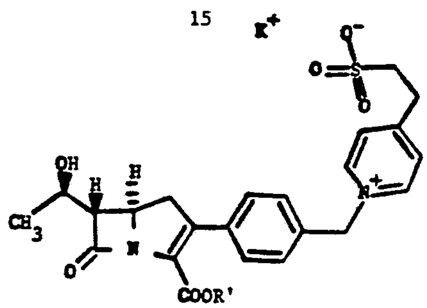

17

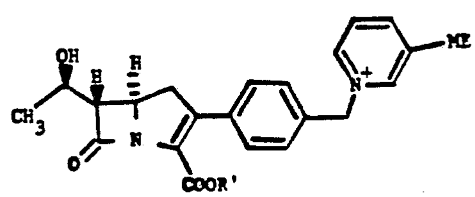

18

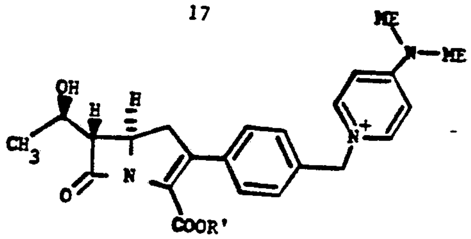

19

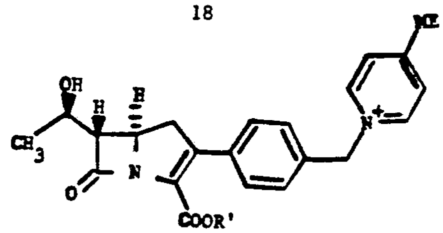

20

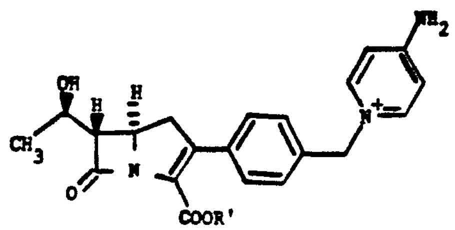

21

ET = ethyl   ME = methyl   PH = phenyl

where R′ is a negative charge $^{\ominus}$, H, a pharmaceutically acceptable ester or salt of the acid, or additionally a readily removable carboxyl covering group which is not a pharmaceutically acceptable ester.

Compounds of the present invention may be tautomeric, as is evident from the structure depicted in formula (10) above. Both isomeric forms of such a tautomeric compound are intended to be included within the scope of the present invention and therefore are contemplated to be within the structure of Formula (I).

The carbapenem compounds of the present invention are useful per se and in their pharmaceutically acceptable salt and ester forms in the treatment of bacterial infections in animal and human subjects. Conveniently, pharmaceutical compositions may be prepared from the active ingredients in combination with pharmaceutically acceptable carriers. Thus, the present invention is also concerned with pharmaceutical compositions and methods of treating bacterial infections utilizing as an active ingredient the novel carbapenem compounds of the present invention.

The pharmaceutically acceptable salts referred to above include non-toxic acid addition salts. The Formula I compounds can be used in the form of salts derived from inorganic or organic acids. Included among such salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides aralkyl halides like benzyl and phenethyl bromides and others. Water or oil-soluble or dispersible products are thereby obtained.

The pharmaceutically acceptable esters of the novel carbapenem compounds of the present invention are such as would be readily apparent to a medicinal chemist, and include, for example, those described in detail in U.S. Pat. No. 4,309, 438, Column 9, line 61 to Column 12, line 51, which is incorporated herein by reference. Included within such pharmaceutically acceptable esters are those which are hydrolyzed under physiological conditions, such as pivaloyloxymethyl, acetoxymethyl, phthalidyl, indanyl and methoxymethyl, and those described in detail in U.S. Pat. No. 4,479,947, which is incorporated herein by reference.

The novel carbapenem compounds of the present invention may also take the form where Y is $COOR^3$, where $R^3$ is a readily removable carboxyl covering group. Such conventional covering groups consist of known ester groups which are used to protectively cover the carboxyl group during the synthesis procedures described further below. These conventional covering groups are readily removable, i.e., they can be removed, if desired, by procedures which will not cause cleavage or other disruption of the remaining portions of the molecule. Such procedures include chemical and enzymatic hydrolysis, treatment with chemical reducing agents under mild conditions, and catalytic hydrogenation. Examples of such ester protecting groups include benzhydryl, p-nitrobenzyl, 2-naphthylmethyl, allyl, benzyl, trichloroethyl, silyl such as trimethylsilyl, phenacyl, p-methoxybenzyl, acetonyl, o-nitrobenzyl, 4-pyridylmethyl, and $C_1$-$C_6$ alkyl such as methyl, ethyl or t-butyl.

The compounds of the present invention are valuable antibacterial agents active against various Gram-positive and Gram-negative bacteria and accordingly find utility in human and veterinary medicine. Representative pathogens which are sensitive to the antibacterial agents of the present invention include various species of the following: Staphylococcus, Enterococcus, Escherichia, Klebsiella, Enterobacter, Bacillus, Salmonella, Pseudomonas, Serratia and Proteus. The antibacterials of the invention are not limited

to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration --the parenteral route by injection being preferred for generalized infections. Such matters, however, are left to the routine discretion of the therapist according to principles of treatment well known in the antibacterial art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention.

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg ot 1000 mg. In parenteral administration, the unit dosage is usually the pure compound I in sterile water solution or in the form of a soluble power intended for solution.

The preferred method of administration of the Formula I antibacterial is parenteral by i.v. infusion, i.v. bolus, or i.m. injection.

For adults, 5-50 mg of Formula I antibacterial per kg of body weight given 2, 3, or 4 times per day is preferred. Preferred dosage is 250 mg to 1000 mg of the Formula I antibacterial given two (b.i.d.) three (t.i.d.) or four (q.i.d.) times per day. More specifically, for mild infections, and particularly urinary tract infections, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 mg t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibacterial compounds of Formula I are of the broad class known as carbapenems or 1-carbadethiapenems. Certain of these carbapenems are susceptible to attack by a renal enzyme known as dehydropeptidase (DHP). This attack or degradation may reduce the efficacy of the carbapenem antibacterial agent. Inhibitors of DHP and their use with carbapenem antibacterial agents are disclosed in the prior art [see European Patent Applications No. 79102616.4 filed July 24, 1979 (Patent No. 0 010 573); 79102615.6, filed July 24, 1979 (Patent No. 0 007 614); and No. 82107174.3, filed August 9, 1982 (Publication No. 0 072 014)].

The compounds of the present invention may, where DHP inhibition is desired or necessary, be combined or used with the appropriate DHP inhibitor as described in the aforesaid patents and published application. Thus, to the extent that the cited European patent applications 1.) define the procedure for determining DHP susceptibility of the present carbapenems and 2.) disclose suitable inhibitors, combination compositions and methods of treatment, they are incorporated herein by reference. A preferred weight ratio of Formula I compound:DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

These combination compositions and their use are further embodiments of the present invention.

## DETAILED DESCRIPTION OF THE INVENTION

The 2-(heteroaryliumalkyl)phenyl carbapenem compounds of the present invention may be prepared in accordance with well known procedures in the art. Particularly useful are the following synthetic schemes in which the symbols R, $R^1$, $R^2$, $R^a$,

$R^b$, $R^c$, A and 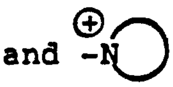 are as defined above.

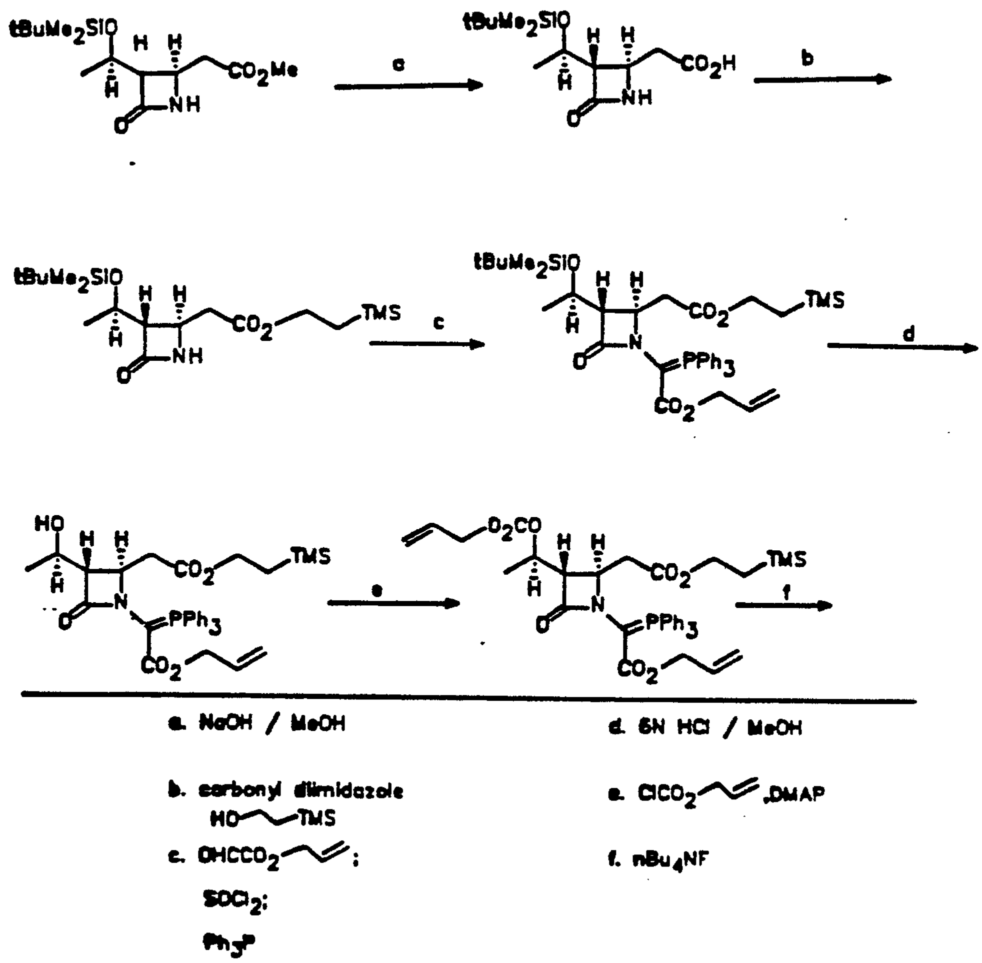

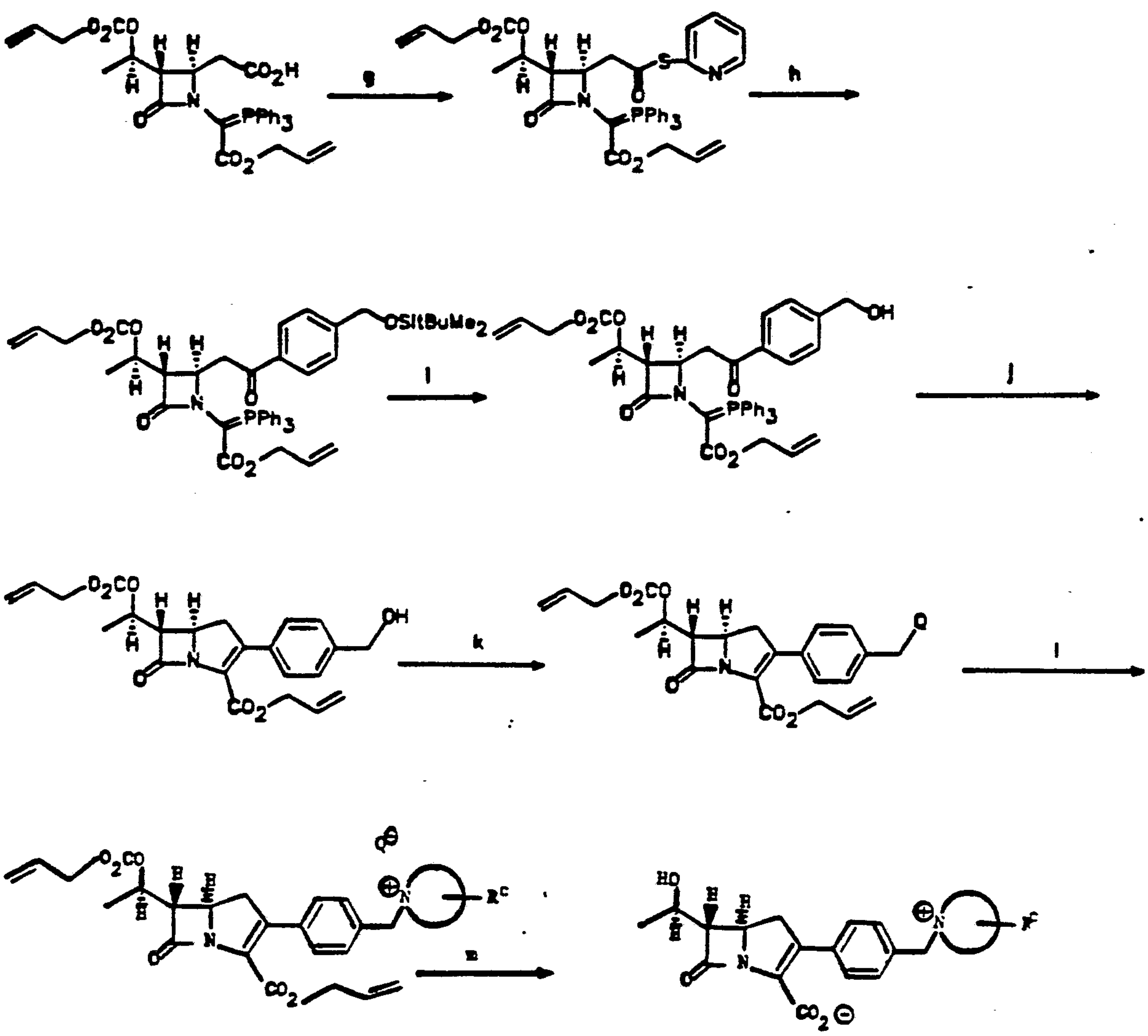


g. Pyr–SS–Pyr, $Ph_3P$

h $BrMg-Ph-CH_2OSi(Me)_2t-Bu$

i. $H_2SO_4$ / MeOH

j. xylenes, 145°C

k. activate (Q=–OMs, –OTf, –I)

l. 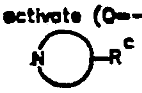

m. $(Ph_3P)_4Pd$, $Ph_3P$
$C_7H_{15}CO_2H$
$C_7H_{15}CO_2K$

The steps for preparing the 2-(p-hydroxymethylphenyl)carbapenem intermediate are well known in the art and are explained in ample detail in U.S. Pat. Nos. 4,260,627 and 4,543,257, which are incorporated herein by reference. Addition of the heteroaryliumalkyl moiety, generally represented by the formula:

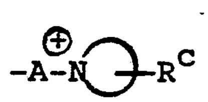

, is as represented in the schematic diagram above. The bridging element "A" is already in place when the phenyl group becomes a part of the carbapenem compound at the time of cyclization. The bridging element terminates in a hydroxyl group which is then changed to an active leaving group, e.g., iodide. Treatment with the desired heteroaryl reactant directly provides the heteroaryliumalkylphenyl sidechain. More particularly, three alternative procedures may be utilized for addition of the heteroarylium group.

## Activation of the 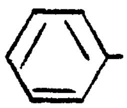-A-OH Group:

This step may be carried out in accordance with well-known procedures, some of which are exemplified in the following equations. In words relative to the equations, the hydroxyl group may be

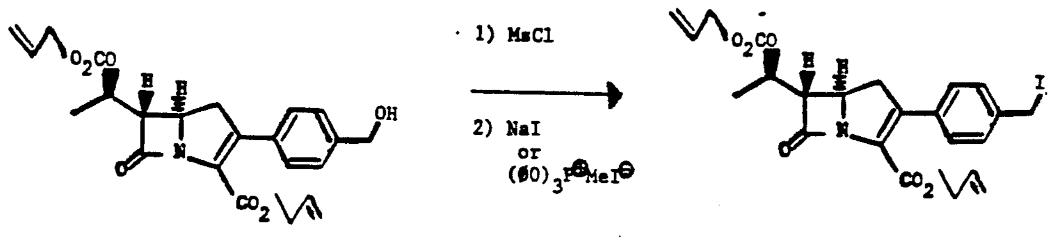


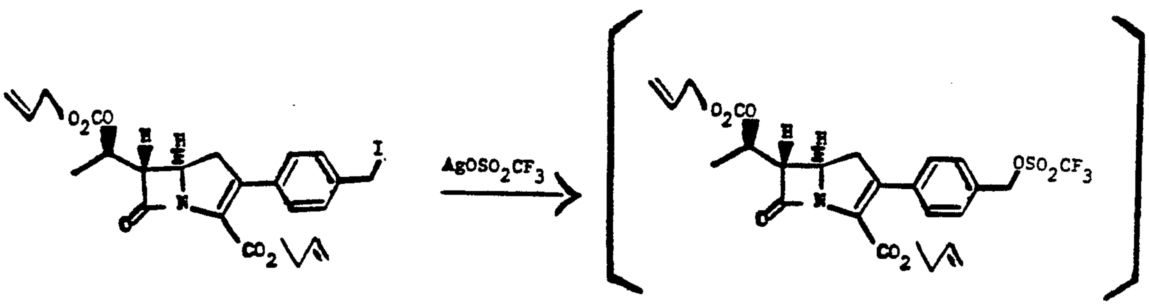


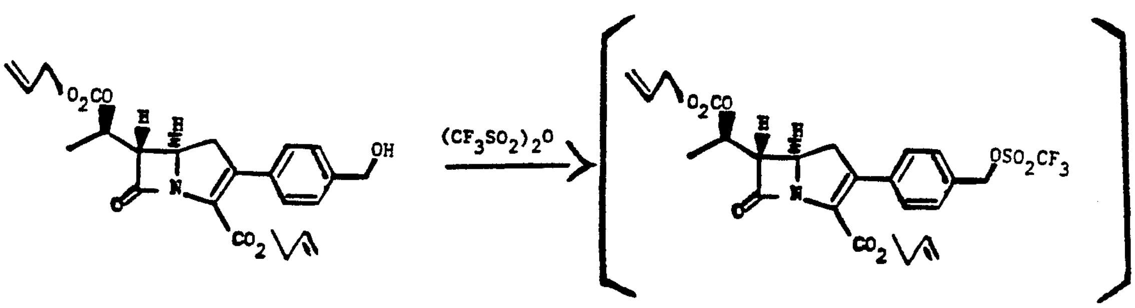


converted to a methanesulfonate group by treating with methanesulfonyl chloride in the presence of triethylamine. A suitable solvent, e.g., dichloromethane, is employed and the reaction is carried out at reduced temperatures. In turn, the methanesulfonate intermediate may be converted to the more reactive iodide derivative by treatment with sodium iodide in a suitable solvent, e.g., acetone, at reduced or ambient temperatures. Alternatively, the hydroxy group may be directly converted into the iodide group by common methods known in the art. For example, treatment of the hydroxyl group with methyl triphenoxyphosphonium iodide in a suitable solvent, such as dimethylformamide, at reduced or ambient temperatures, directly provides the desired iodide. Further, the hydroxyl group may be converted into the very reactive trifluoromethanesulfonate group. However, such an activating group cannot be isolated by conventional techniques but may be formed and used in situ. Thus, treatment of the hydroxyl group with trifluoromethanesulfonic acid anhydride in the presence of, usually, the reacting heteroaromatic base in a suitable solvent, such as dichloromethane, at reduced temperatures provides for the in situ generation of this activating group. Alternatively, the trifluoromethanesulfonate group may be generated in situ from the iodide group by treatment with excess silver trimethanesulfonate in a suitable solvent, e.g., acetonitrile, at reduced temperatures.

Once the desired activation has been carried out, introduction of the heteroarylium group can then proceed. One of the following three procedures as been found suitable for such introduction.

Method A:

The activated group is iodide and the addition of the heteroarylium group, e.g., pyridinium, is accomplished simply by treating with the corresponding heteroaryl, e.g., pyridine, in a suitable solvent, e.g., acetonitrile, at about room temperature.

Method B:

The activating group is trifluoromethanesulfonate and is formed in situ by treatment of the alcohol with trifluoromethanesulfonic acid anhydride in the presence of at least two equivalents of heteroaryl to provide the corresponding heteroarylium in a suitable solvent, e.g., dichloromethane, at reduced temperatures.

Method C:

The activated group is trifluoromethanesulfonate which is formed in situ by treatment of the iodide derivative with excess silver trifluoromethanesulfonate in a suitable solvent, e.g., acetonitrile, at reduced temperatures. As with Method A, the heteroaryl to provide the corresponding heteroarylium is simply added and displacement of the activating group then takes place directly.

Where the heteroarylium group has a substituent $R^c$, the most facile method of providing such a substituent is to employ as the reactant in the preparation methods described above a heteroaryl compound which already has the desired substituent. Such substituted heteroaryl compounds are readily available starting materials or may be prepared in a straight-forward manner using known literature methods.

In the preparation methods described above, the carboxyl group at the 3-position remains blocked by a carboxyl covering group until the final product is prepared. Then, if the anionic carboxylate is desired so as to form the zwitterionic internal salt, deblocking may be carried out in a conventional manner, with care being taken to avoid a procedure which is so harsh as to disrupt other portions of the final product molecule.

EXAMPLE 1

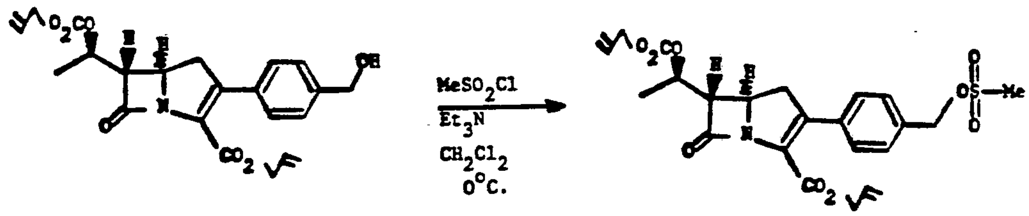


1 2

To a stirred solution of 42.7 mg (0.1 mmole) of 1 in 1 ml of sieve dried $CH_2Cl_2$ at 0°C under a nitrogen atmosphere was added sequentially 15.2 mg (0.15 mmole) of neat $Et_3N$ and then 14.9 mg (0.13 mmole) of neat mesyl chloride. The resulting mixture was stirred for 15 minutes, and then partitioned between EtOAc, ice-$H_2O$, and some 2N HCl. The organic phase was separated, washed with saturated NaCl solution, dried over $Na_2SO_4$, filtered, evaporated, and dried in vacuo to give a quantitative yield of 2; IR ($CH_2Cl_2$): 1780, 1745, 1725 $cm^{-1}$; 200 MHz $^1$H-NMR ($CDCl_3$): $\delta$ 1.49 (d, J=6.4 Hz, $CH_3CH$), 2.96 (s, $CH_3SO_3$), 3.18 (dd, J=9.9, 18.1 Hz, H-1), 3.34 (dd, J=8.9, 18.1 Hz, H-1), 3.43 (dd, J=2.8, 8.1 Hz, H-6), 4.30 (dt, J=2.3, 2.8, 9.9 Hz, H-5), 4.66 (m, $CH_3CHOH$ and $CH_2CH=CH_2$), 5.26 (m, $OCH_2CH=CH_2$), 5.29 (s, $ArCH_2OSO_2$), 7.40 (s, Ar-H).

$$UV: \lambda^{p\text{-diox}}_{max} = 314 \text{ nm.}$$

EXAMPLE 2

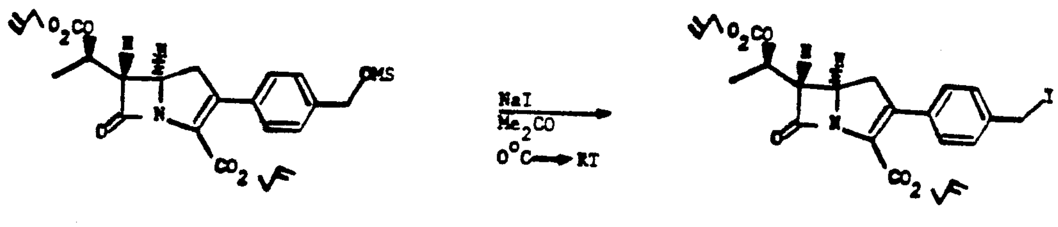


2 3

To a stirred solution of 38.8 mg (0.077 mmole) of 2 in 1 ml of acetone at 0°C was added all at once 23 mg (0.15 mmole) of NaI. The ice-$H_2O$ bath was removed and the mixture stirred further under a nitrogen atmosphere for 0.5 hour. After this time, the resulting mixture was partitioned between EtOAc, ice-$H_2O$, 5% $Na_2S_2O_4$ (aq.) solution and saturated NaCl solution. The organic phase was separated, dried over $Na_2SO_4$, filtered, evaporated and dried in vacuo to give 3; IR ($CH_2Cl_2$): 1780, 1745, 1725 $cm^{-1}$; 200 MHz $^1$H-NMR ($CDCl_3$): δ 1.49 (d, J=7.4 Hz, $C\underline{H}_3$), 3.17 (dd, J=9.8, 18.1 Hz, H-1), 3.29 (dd, J=8.7, 18.1 Hz, H-1), 3.41 (dd, J=2.9, 8.7 Hz, H-6), 4.27 (dt, J=2.9, 8.7, 9.8 Hz, H-5), 4.65 (m, $CH_3C\underline{H}OH$ and $OC\underline{H}_2CH=CH_2$), 5.26 (m, $OCH_2CH=C\underline{H}_2$), 5.89 (m, $OCH_2C\underline{H}=CH_2$), 7.32 (m, Ar-H).

$$\text{UV}\cdot\ \lambda_{max}^{p\text{-}diox} = 322\ nm.$$

METHOD A

EXAMPLE 3

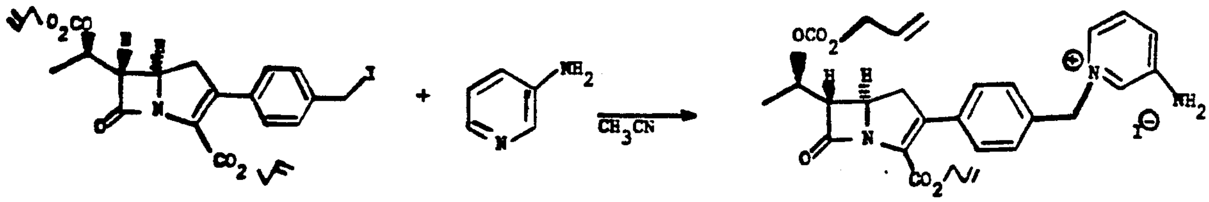


3 4

To a stirred solution of 161 mg (0.299 mmoles) of 3 in 5 ml of anhydrous acetonitrile was added 31.0 mg (0.33 mmoles) of solid 3-aminopyridine. The resulting solution was stirred at room temperature for 17.5 hours under a $N_2$ atmosphere and then partitioned between $CH_2Cl_2$ and ice/$H_2O$. The organic phase was separated, dried with anhydrous $Na_2SO_4$, filtered and concentrated under vacuum to provide 204.5 mg of residue as an orange film. The film was redissolved in 1 ml methylene chloride and the solution triturated with 12 ml $Et_2O$. The gummy solid which separated was collected and dried under vacuum to provide 189 mg (100%) of 4 as an orange foam; IR ($CH_2Cl_2$): 3280, 3160, 1785, 1745, 1725 $cm^{-1}$; 300 MHz $^1$H-NMR ($CDCl_3$): δ 1.47 (d, J=5.8 Hz, $C\underline{H}_3CH$), 3.17 (dd, J=9.8, 19 Hz, H-1), 3.37 (dd, J=8.8, 19 Hz, H-1), 3.57 (dd, J=2.8, 7.4 Hz, H-6), 4.30 (dt, J=2.0, 9.8 Hz, H-5), 4.63 (d, J=6.2, $OC\underline{H}_2CH=CH_2$), 4.65 (m, H-8), 5.25 (m, $OCH_2CH=C\underline{H}_2$), 5.61 (s, $ArC\underline{H}_2$), 5.88 (m, $CH_2C\underline{H}=CH_2$), 6.29 (s, $N\underline{H}_2$), 7.41 (d, J=8.3 Hz, Ar-H), 7.42 (t, pyridine-H-5), 7.49 (d, J=8.3 Hz, Ar-H), 7.63 (dd, J=1.5, 8.8 Hz, pyridine-H-3), 7.61 (d, J=5.5 Hz, pyridine-H-6), 8.98 (s, pyridine-H-2);

$$\text{UV:}\ \lambda_{max}^{diox} = 307,\ 268\ (s)\ nm.$$

EXAMPLE 4

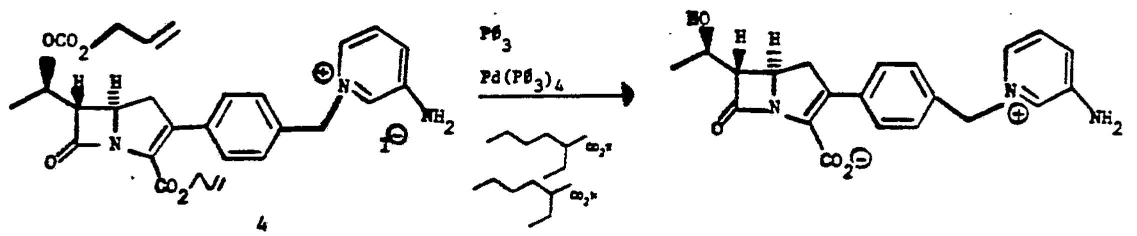


4

5

To a stirred solution of 157 mg (0.249 mmoles) of 4 in 5 ml anhydrous $CH_2Cl_2$ and 1.5 ml anhydrous EtOAc was added a mixture of 39.2 mg (0.149 mmoles) triphenylphosphine and 57.5 mg (0.0498 mmoles) of tetrakis (triphenylphosphine) palladium, followed by 43.7 μl (0.274 mmoles) of 2-ethyl hexanoic acid and 548 μl (0.274 mmoles) of a 0.5M potassium 2-ethyl hexanoate in EtOAc. The mixture was stirred at room temperature for 5 hours under an $N_2$ atmosphere. A light colored precipitate developed after 10 minutes. The reaction slurry was then diluted with EtOAc and the solid separated and washed 3 times with $Et_2O$. The solid was then dried in vacuo and purified by reversed phase-PLC (3 x 1000 μ, 20 x 20 cm, reverse phase silica gel F plates, eluted at 5° with 30% THF in $H_2O$). The major UV active product bands were combined and extracted 8 times with $CH_3CH-H_2O$ (4:1). The combined aqueous extracts were washed 3 times with hexanes, filtered through a Gelman Acrodisc-CR 0.45 μ filter assembly and concentrated under vacuum. The concentrate was lyophilized to give 50.3 mg of 5 as an off white fluffy solid; IR (nujol mull): 3300, 3150, 1750, 1580 cm$^{-1}$; 200 MHz $^1$H-NMR ($D_2O$): δ 1.31 (d, J=6.3 Hz, $CH_3CH$), 3.13 (dd, J=9.8, 16.8 Hz, H-1), 3.41 (dd, J=8.57, 16.8 Hz, H-1), 3.50 (dd, J=2.3, 5.4 Hz, H-6), 4.38 (m, H-8 and H-5), 5.58 (s, $PhCH_2$), 7.39 (m, Ar-H and pyridine-H-5), 7.67 (m, pyridine-H-4), 8.10 (m, pyridine-H-2 and H-6);

$$\text{UV: } \lambda_{max}^{H_2O} = 306, 254 \text{ nm.}$$

METHOD B

EXAMPLE 5

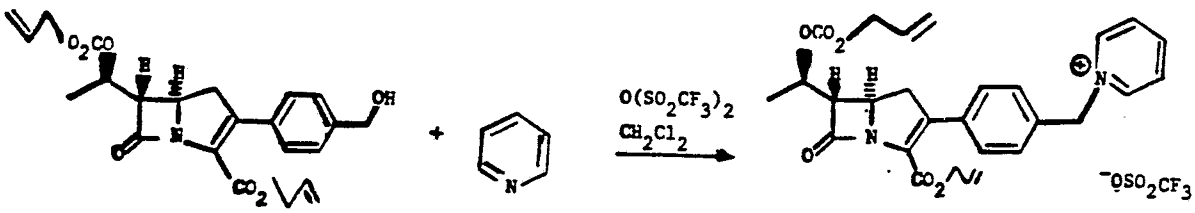


1

6

To a stirred solution of 117.4 mg (0.275 mmoles) of 1 in 5 ml anhydrous $CH_2Cl_2$ at -20°C under a $N_2$ atmosphere was added 55.6 μl (0.687 mmoles) of pyridine, then 60.1 μl (0.357 mmoles) of trifluoromethanesulfonic anhydride. The resulting solution was stirred 2 hours at -20°C and then partitioned between methylene chloride and ice/$H_2O$. The organic phase was separated, washed with saturated aqueous NaCl, dried with $Na_2SO_4$, filtered and concentrated under vacuum to provide 177.1 mg of a residue as a yellow film. The crude reaction residue was dissolved in methylene chloride and the solution triturated with ether. The gummy solid which separated was collected and dried under vacuum to provide 164.4 mg (94%) of 6, as a yellow foam; IR ($CH_2Cl_2$): 1785, 1745, 1725 cm$^{-1}$; 200 MHz NMR ($CDCl_3$): δ 147 (d, J=6.4 Hz, $CH_3CH$), 3.14 (dd, J=10.3, 18.5 Hz, H-1), 3.31 (dd, J=9.0, 18.5 Hz, H-1), 3.46 (dd, J=2.9, 7.9 Hz, H-6), 4.32 (dt, J=2.9, 9.8 Hz, H-5), 4.65 (m, H-8 and $CH_2CH=CH_2$), 5.25 (m, $CH_2CH=CH_2$), 5.87 (s, Ar-$CH_2$), 5.87 (m, $CH_2CH=CH_2$), 7.40 (d, J=8.2 Hz, Ar-H), 7.51 (d, J=8.2 Hz, Ar-H), 7.97 (t, J=6.7 Hz, pyridine-H-3 and H-5), 8.42 (t, J=6.5 Hz, pyridine-H-4), 9.06 (d, J=5.3 Hz, pyridine-H-2 and H-6;

$$UV: \lambda_{max}^{diox} = 316,266 \text{ nm.}$$

METHOD C

EXAMPLE 6

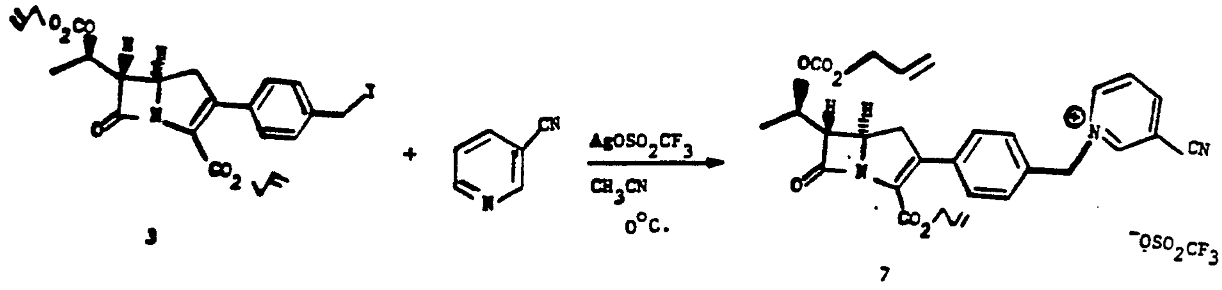


3 + → 7

To a stirred solution of 58.8 mg (0.109 mmoles) of 3 in 1.5 ml anhydrous acetonitrile at 0°C under a $N_2$ atmosphere was added a solution of 14.8 mg (0.142 mmoles) of nicotinonitrile in 200 µl anhydrous acetonitrile, followed by a solution of 64 mg (0.25 mmoles) of silver trifluoromethanesulfonate in 350 µl anhydrous acetonitrile. The resulting yellow slurry was stirred 1.5 hours at 0°C then concentrated under vacuum. The residue was dissolved in $CH_2Cl_2$ and the mixture filtered through a Gelman Acrodisc-CR 0.45 µ filter assembly. The yellow filtrate was evaporated under vacuum to provide 72.4 mg of a residue, as a yellow film. The crude residue was dissolved in 1 ml methylene chloride and the solution triturated with 11 ml $Et_2O$. The solid which separated was collected and redissolved in $CH_2Cl_2$. The ether trituration was repeated twice and then the collected solid dried under vacuum to provide 45.1 mg (62%) of 7: IR ($CH_2Cl_2$): 1770, 1740, 1725 $cm^{-1}$; 200 MHz NMR ($CDCl_3$): δ 1.48 (d, J-6.1 Hz, $CH_3CH$), 3.14 (dd, J=10.2, 18.4 Hz, H-1), 3.35 (dd, J=8.5, 18.4 Hz, H-1), 3.49 (dd, J=2.5, 7.6 Hz, H-6), 4.28 (m, H-5), 4.65 (m, H-8 and $CH_2CH=CH_2$), 5.28 (m, $CH_2CH=CH_2$), 5.9 (m, $CH_2CH=CH_2$), 6.05 (s, Ar-$CH_2$), 7.42 (d, J=8.2 Hz, Ar-H and pyridine H-4), 7.56 (d, J=8.2 Hz, Ar-H), 8.33 (t, J=7.0 Hz, pyridine-H-5), 8.73 (d, J=7.6 Hz, pyridine-H-6), 9.32 (s, pyridine-H-2).

EXAMPLES 7-31

Employing the procedures described above, additional compounds of the present invention were prepared. These are described in the table below, which additionally includes characterizing data and the method of preparation for each compound.

## TABLE I

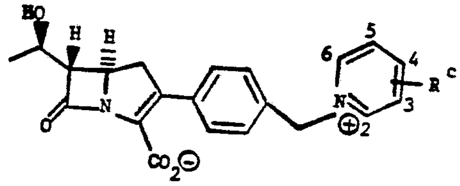

| Example No. | $R^C$ | $\lambda_{max}^{H_2O}$ (nm) | Method of Preparation |
|---|---|---|---|
| 7 | H | 257, 303 | A or B |
| 8 | $2\text{-}NH_2$ | 303 | A |
| 9 | $3\text{-}NH_2$ | 308 | A |
| 10 | $4\text{-}NH_2$ | 274, 301 | A |
| 11 | $2\text{-}NHCH_3$ | 307 | A |
| 12 | $2\text{-}N(CH_3)_2$ | 306 | B |
| 13 | $4\text{-}N(CH_3)_2$ | 300 | A or B |
| 14 | $3\text{-}\underset{H}{N}CH_2CO_2K$ | 267, 306 | A |
| 15 | $4\text{-}\underset{H}{N}CH_2CO_2K$ | 287 | A |
| 16 | $3\text{-}CO_2K$ | 305 | B |
| 17 | $3\text{-}CON(CH_3)_2$ | 304 | B |
| 18 | 3-CN | 305 | C |
| 19 | $3\text{-}CF_3$ | 304 | C |
| 20 | $3\text{-}SCH_3$ | 228, 274, 306 | A |
| 21 | 4-Ø | 300 | B |
| 22 | $2\text{-}CH_3$ | 267, 304 | A or B |
| 23 | $3\text{-}CH_3$ | 304 | A or B |
| 24 | $4\text{-}CH_3$ | 304 | A or B |
| 25 | $2\text{-}CH_2CH_3$ | 268, 304 | B |
| 26 | $4\text{-}CH_2NH_2$ | 304 | A |
| 27 | $4\text{-}CH_2SO_3K$ | 304 | A |
| 28 | $4\text{-}CH_2CH_2SO_3K$ | 304 | A |
| 29 | $3\text{-}CH_2SCH_3$ | 304 | A |
| 30 | $3\text{-}CH_2SCH_2CH_2CN$ | 303 | A |
| 31 | $3\text{-}CH_2SCH_2CF_3$ | 303 | A |

EXAMPLE 32

Following the procedures described above, additional compounds of the present invention may be prepared, as set forth in the following table.

TABLE II

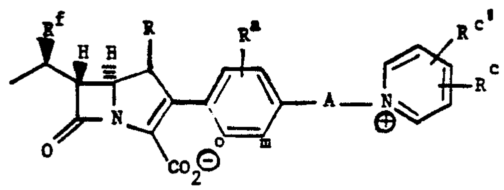

| $R^f$ | R | $R^a$ | $R^c$ | A | $R^{c'}$ |
|---|---|---|---|---|---|
| -OH | H | H | $2-NH_2$ | $-CH_2CH_2-$ | H |
| -OH | H | H | $3-CH_2SCH_3$ | $-SCH_2CH_2-$ | H |
| -OH | H | H | $4-NH_2$ | $-CH_2SCH_2-$ | H |
| -F | H | H | $3-CH_2SCH_3$ | $-CH_2-$ | H |
| -F | $CH_3$ | H | $3-SCH_3$ | $-CH_2-$ | H |
| -OH | H | H | 3-F | $-CH_2-$ | H |
| -OH | H | $m-CH_3$ | $2-NH_2$ | $-CH_2-$ | H |
| -OH | H | m-F | $3-CH_2SCH_3$ | $-CH_2-$ | H |
| -OH | H | m-OH | $3-CH_2SCH_3$ | $-CH_2-$ | H |
| -OH | $CH_3$ | H | $2-NH_2$ | $-CH_2-$ | H |
| -OH | H | H | $4-NH_2$ | $-CH_2-$ | $3-CH_2SMe$ |
| -OH | H | H | $3-CH_2CO_2K$ | $-CH_2-$ | $5-CH_2COOK$ |
| -OH | H | H | $3-CO_2K$ | $-CH_2-$ | 4-OH |

## Claims

1. A compound of the formula:

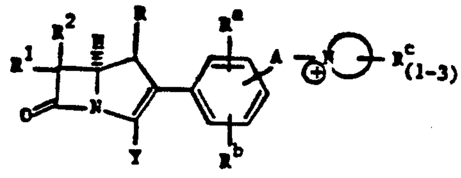

(I.)

wherein:
R is H or $CH_3$;
$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$__, $FCH_2$-, $F_2CH$-, $F_3C$-, $CH_3CH(F)$-, $CH_3CF_2$-, or $(CH_3)_2C(F)$-;
$R^a$, $R^b$ and $R^c$ ($R^c$ represents from 1 to 3 substituents which may be the same or different) are independently selected from the group consisting of:

a) a trifluoromethyl group: $-CF_3$;
b) a halogen atom: -Br, -Cl-, -F, or -I;
c) $C_1$-$C_4$ alkoxy radical: $-OC_{1-4}$ alkyl;
d) a hydroxy group: -OH;
e) ($C_1$-$C_6$ alkyl) carbonyloxy radical:
$-O\overset{O}{\overset{\parallel}{C}}\ C_{1-6}$ alkyl;
f) a carbamoyloxy radical which is unsubstituted or substituted on nitrogen with one or two $C_1$-$C_4$ alkyl groups:

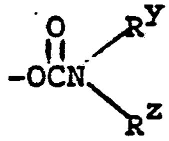

where $R^y$ and $R^z$are independently H or $C_{1-4}$ alkyl;
g) a $C_1$-$C_6$ alkylthio radical,
$C_1$-$C_6$ alkylsulfinyl radical or $C_1$-$C_6$ alkylsulfonyl radical:

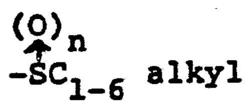

where n = 0-2, and the alkyl portion is optionally substituted by cyano;
h) a sulfamoyl group which is unsubstituted or substituted on nitrogen by one or two $C_1$-$C_4$ alkyl groups:

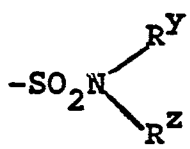

where $R^y$ and $R^z$ are as defined above;
i) an amino group, or a mono ($C_1$-$C_4$ alkyl) amino or di($C_1$-$C_4$ alkyl)-amino group:

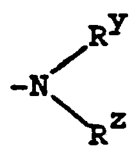

where $R^y$ and $R^z$ are as defined above;
j) a formylamino group:

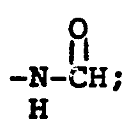

k) ($C_1$-$C_6$ alkyl)carbonylamino radical:

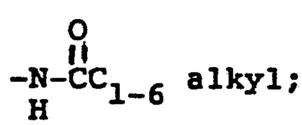

l) a ($C_1$-$C_4$ alkoxy) carbonylamino radical:

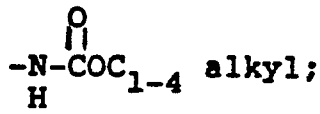

m) a ureido group in which the terminal nitrogen is unsubstituted or substituted with one or two $C_1$-$C_4$ alkyl groups:

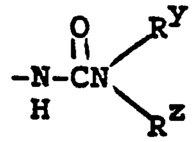

where $R^y$ and $R^z$ are as defined above;
n) a sulfonamido group: $-\underset{H}{N}SO_2$;
o) a cyano group: -CN;
p) a formyl or acetalized formyl radical:

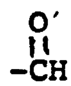 or 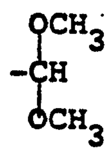 ;

q) ($C_1$-$C_6$ alkyl)carbonyl radical wherein the carbonyl is free or acetalized:
$-\overset{O}{\overset{\|}{C}}\ C_{1-6}$ alkyl or

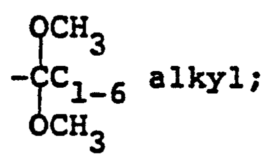

r) phenylcarbonyl: $-\overset{O}{\overset{\|}{C}}-$;
s) a hydroximinomethyl radical in which the oxygen or carbon atom is optionally substituted by a $C_1$-$C_4$ alkyl group:
$-\overset{R^y}{\overset{|}{C}}=NOR^z$ where $R^y$ and $R^z$ are as defined above;
t) a ($C_1$-$C_6$ alkoxy)carbonyl radical:
$-\overset{O}{\overset{\|}{C}}OC_{1-6}$ alkyl;
u) a carbamoyl radical which is unsubstituted or substituted on nitrogen by one or two $C_1$-$C_4$ alkyl groups:

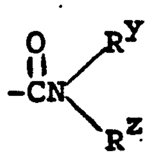

where $R^y$ and $R^z$ are as defined above;
v) an N-hydroxycarbamoyl or N($C_1$-$C_4$ alkoxy)carbamoyl radical in which the nitrogen atom may be additionally substituted by a $C_1$-$C_4$ alkyl group:

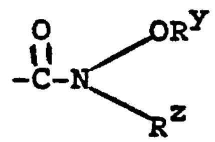

where $R^y$ and $R^z$ are as defined above;

w) a thiocarbamoyl group: - $\overset{S}{\overset{\|}{C}}NH_2$;

x) an amidino group

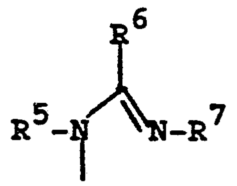

or

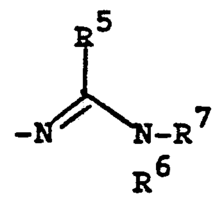

where $R^5$, $R^6$ and $R^7$ are independently hydrogen, $C_1$-$C_4$alkyl or wherein two of the alkyl groups together form a $C_2$-$C_6$alkylidene radical optionally interrupted by a heteroatom and joined together to form a ring;

y) a carboxamidino group

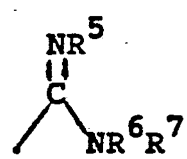

where $R^5$, $R^6$ and $R^7$ are as defined above;

z) a guanidinyl group where $R^6$ in y) above is $NR^8R^9$ and $R^8$ and $R^9$ are as defined for $R^5$ through $R^7$ above;

aa) hydrogen;

ab) $C_2$-$C_6$ alkenyl radical;

ac) $C_2$-$C_6$ alkynyl radical;

ad) $C_3$-$C_7$ cycloalkyl radical;

ae) $C_3$-$C_7$ cycloalkyl methyl radical;

af) $C_5$-$C_7$ cycloalkyl radical;

ag) phenyl;

ah) $C_1$-$C_6$ alkyl radical;

ai) $C_1$-$C_4$ alkyl monosubstituted by one of the substituents a) - ag) above;

aj) an acidic sidechain of the structure -B or $-(CH_2)_n-X-(CH_2)_m-W-B$ where:

n is 0-4;

m is 0-4;

X is $CR^sR^t$; CH = CH; phenylene ($-C_6H_4-$); NH; N($C_1$-$C_4$ alkyl); O; S; S = O; C = O; $SO_2$; $SO_2NH$; $CO_2$; CONH; $OCO_2$; OC = O; or NHC = O;

where $R^t$ is H or $C_1$-$C_4$-alkyl; and $R^s$ is H, OH, $C_1$-$C_4$-alkyl, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, or $SO_2NH$($C_1$-$C_4$ alkyl);

W is a single bond; NH; N($C_1$-$C_4$ alkyl); O; or S;

B is an acidic function selected from carboxy ($CO_2H$); phosphono [P = O(OH)$_2$]; alkylphosphono {P = O(OH)-[O($C_1$-$C_4$ alkyl)]}; alkylphosphinyl [P = O(OH)-($C_1$-$C_4$ alkyl)]; phosphoramido [P = O(OH)$NH_2$]; substituted phosphor amido [P = O(OH)NH($C_1$-$C_4$ alkyl) and P = O(OH)$NHR^x$]; sulfino ($SO_2H$); sulfo ($SO_3H$); 5-tetrazolyl ($CN_4H$); arylsulfonamido ($SO_2NHR^x$); and acylsulfonamides selected from the structures $CONHSO_2R^x$, $CONHSO_2NH_2$, $CONHSO_2$($C_1$-$C_4$ alkyl), $CONHSO_2NH$($C_1$-$C_4$ alkyl), $CONHSO_2N$($C_1$-$C_4$ alkyl)$_2$, $SO_2NHCO$($C_1$-$C_4$ alkyl), $SO_2NHCONH_2$, $SO_2NHCONH$($C_1$-$C_4$ alkyl), $SO_2NHCON$($C_1$-$C_4$ alkyl)$_2$, $SO_2NHCOR^x$, $SO_2NHCSNH_2$, $SO_2NHCSNH$($C_1$-$C_4$ alkyl), $SO_2NHCSN$($C_1$-$C_4$ alkyl)$_2$, $SO_2NHSO_2R^x$, and $SO_2NHSO_2R^w$, where $R^x$ is phenyl or heteroaryl; and $R^w$ is $C_1$-$C_4$alkyl, phenyl, heteroaryl, $NH_2$, NH($C_1$-$C_4$ alkyl), N($C_1$-$C_4$ alkyl)$_2$, OH, or O($C_1$-$C_4$alkyl);

where, for both $R^x$ and $R^w$, heteroaryl is a monocyclic aromatic hydrocarbon group having 5 or 6 ring atoms in which from 1 to 4 carbon ring atoms are replaced by heteroatoms selected from N, O, and S, provided that the minimum number of nitrogen heteroatoms present when 2 to 4 total heteroatoms are selected, is

equal to the total number of heteroatoms less one;
and where for both $R^w$ and $R^x$ the phenyl and heteroaryl groups may be further mono-or disubstituted by $C_1$-$C_4$alkyl, $C_1$-$C_4$-alkoxy, Br, Cl, F, $CF_3$, OH, ($C_1$-$C_4$alkyl)carbonyloxy, carbamoyl or carbamoyloxy or sulfamoyl or amino where the nitrogen may be mono-or disubstituted with $C_1$-$C_4$alkyl, $C_1$-$C_4$alkylthio, $C_1$-$C_4$ alkylsulfinyl, $C_1$-$C_4$alkylsulfoxyl, formylamino, ($C_1$-$C_4$alkyl)carbonylamino, ($C_1$-$C_4$alkoxyl)carbonylamino, ureido in which the terminal nitrogen may be substituted by $C_1$-$C_4$alkyl, ($C_1$-$C_4$alkyl)sulfonamido, cyano, ($C_1$-$C_4$alkoxyl)carbonyl, thiocarbamoyl, nitro, carboxyl, formyl, or ($C_1$-$C_4$alkyl)carbonyl;
A is para (p) or meta (m) with respect to the point of attachment of the phenyl ring to the carbapenem nucleus, and is $(CH_2)_m$-Q-$(CH_2)_n$, where m is 0 to 2 and n is 1 or 2; and Q is a covalent bond; O; S; SO; $SO_2$; NH; or N($C_1$-$C_4$ alkyl);

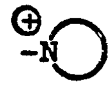

is a monocyclic aromatic hydrocarbon group having 5 or 6 ring atoms in which one of the carbon atoms has been replaced by a nitrogen atom and attachment of said group is by way of said nitrogen atom, and in which one additional carbon atom is optionally replaced by a heteroatom selected from O and S, or from 1 to 3 additional carbon atoms are each optionally replaced by a nitrogen heteroatom; and
Y is selected from: i) COOH or a pharmaceutically acceptable ester or salt thereof,
ii) $COOR^3$ wherein $R^3$ is a readily removable carboxyl covering group,
iii) COOM wherein M is an alkali metal, or
iv) $COO^{\ominus}$;
provided that when Y is other than iv) a counterion $Z^{\ominus}$ is provided.

2. A compound according to Claim 1 wherein the compound is selected from the group consisting of

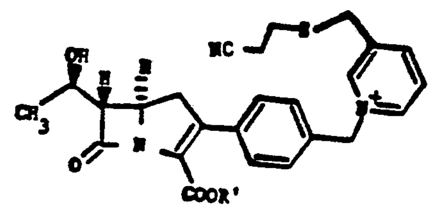

1

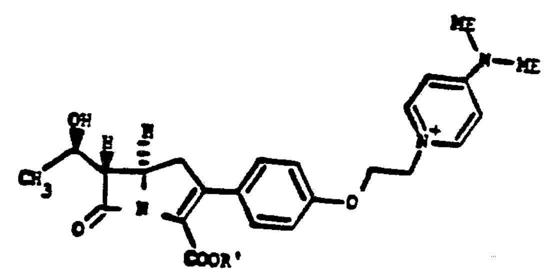

2

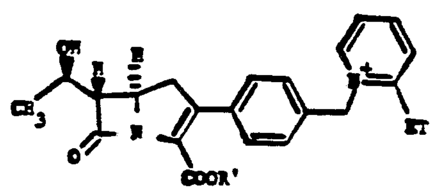

3

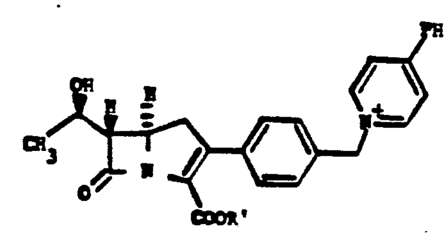

4

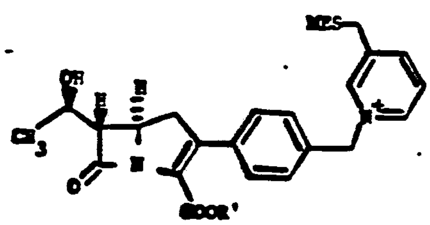

5

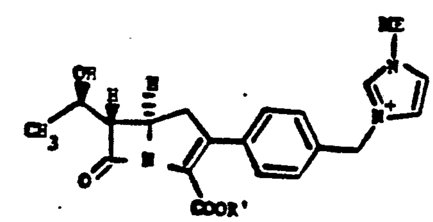

6

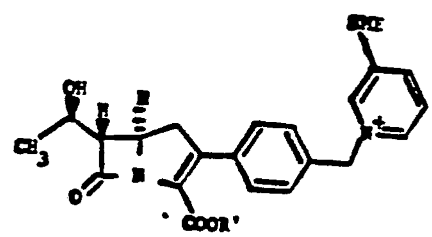

7

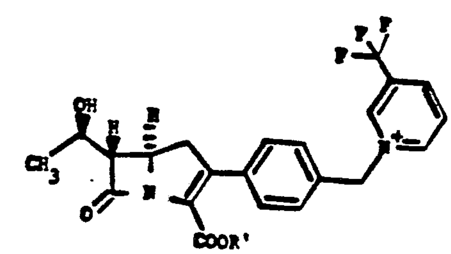

8

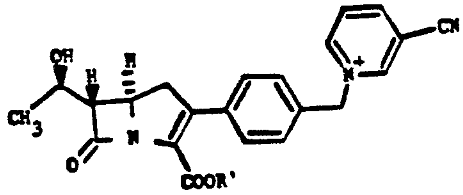

9

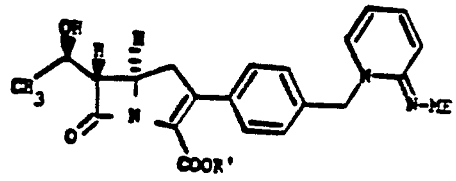

10

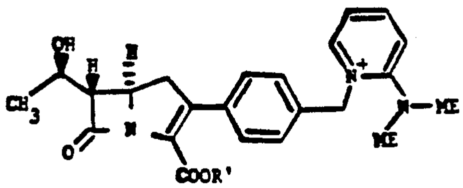

11

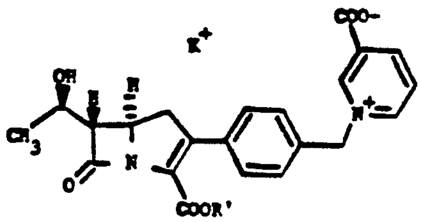

12

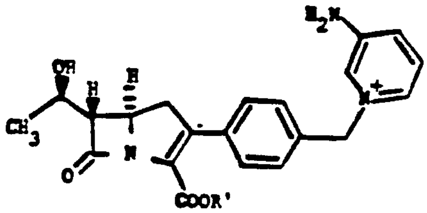

13

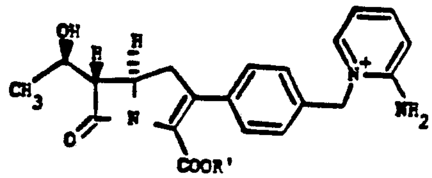

14

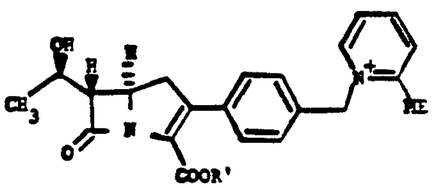

15

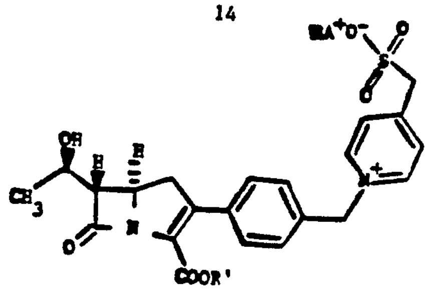

16

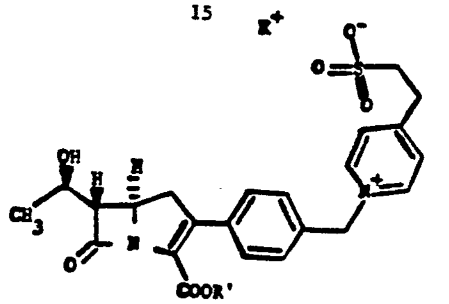

17

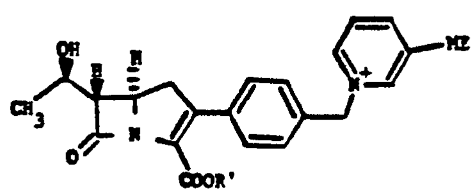

18

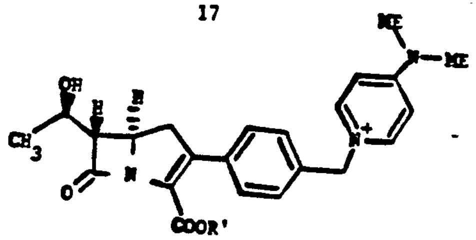

19

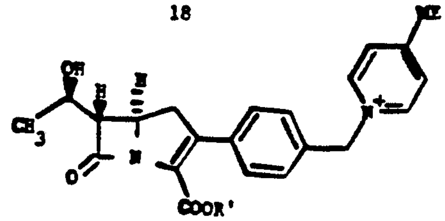

20

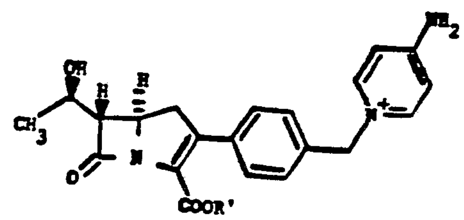

21

ET = ethyl ME = methyl PH = phenyl

where R' is a negative charge $^{\ominus}$, H, a pharmaceutically acceptable ester or salt of the acid, or additionally a readily removable carboxyl protecting group which is not a pharmaceutically acceptable ester.

3. A pharmaceutical composition for antibacterial use comprising an antibacterially effective amount of a compound of Claim 1 and a pharmaceutically acceptable carrier thereof.

4. A method of treating bacterial infections in human or animal subjects in need of such treatment comprising administering to such subject an antibacterially effective amount of a compound of Claim 1.

5. The combination of a compound of Claim 1 and a DHP inhibitor.

6. The combination of a compound of Claim 6 and the DHP inhibitor 7-(L-2-amino-2-carboxyethylthio)-2-(2,2-dimethylcyclopropanecarboxamide)-2-heptenoic acid.

7. A pharmaceutical composition for antibacterial use comprising an antibacterially effective amount of a compound of Claim 1, an inhibitorily effective amount of a DHP inhibitor, and, optionally, a pharmaceutically acceptable carrier thereof.

8. A pharmaceutical composition according to Claim 11 wherein the DHP inhibitor is 7-(L-2-amino-2-carboxyethylthio)-2-(2,2-dimethylcyclopropanecarboxamide)-2-heptenoic acid.

European Patent Office

# PARTIAL EUROPEAN SEARCH REPORT

which under Rule 45 of the European Patent Convention shall be considered, for the purposes of subsequent proceedings, as the European search report

Application number
EP 88 30 0609

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A - 0 184 842 (MERCK)<br>* Claims *<br>-- | 1,3,5-8 | C 07 D 487/04<br>A 61 K 31/40//<br>(C 07 D 487/04,<br>209/00,<br>205/00) |
| A | EP - A - 0 185 315 (MERCK)<br>* Claims *<br>-- | 1,3,5-8 | |
| A | EP - A - 0 010 316 (MERCK)<br>* Pages 49,82-84; claims * | 1,3,5-8 | |
| D | & US - A - 4 260 627<br>------------------- | | |
| | | | TECHNICAL FIELDS SEARCHED (Int. Cl.4) |
| | | | C 07 D 487/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-3,5-8
Claims searched incompletely:
Claims not searched: 4
Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 28-04-1988 | CHOULY |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document